# EUROPEAN PATENT APPLICATION

(11) **EP 0 569 096 A1**
(43) Date of publication of application: **10.11.1993**
(21) Application number: 93201270.1
(22) Date of filing: 04.05.1993
(51) Int. Cl.: C07D 491/044, A61K 9/10, A61K 31/40

(54) **Depot preparation**

(30) Priority: 08.05.1992 EP 92201319
(71) Applicant: Akzo Nobel N.V., NL-6824 BM Arnhem (NL)
(72) Inventor: van den Oetelaar, Petrus Johannes Maria, NL-5384 ET Heesch (NL)
(74) Representative: Beetz, Tom

(57) **Abstract**

Disclosed are the hemipamoate and pamoate salt of trans-5-chloro-2-methyl-2,3,3a,12b-tetrahydro-1H-dibenz-[2,3;6,7]oxepino-[4,5-c]pyrrole, very insoluble compounds having excellent properties for use as depot preparations. Also disclosed are pharmaceutical preparations which include the pamoate salts of trans-5-chloro-2-methyl-2,3,3a,12b-tetrahydro-1H-dibenz[2,3;6,7]oxepino-[4,5-c]pyrrole, especially in crystalline form. Such preparations release therapeutically useful amounts of trans-5-chloro-2-methyl-2,3,3a,12b-tetrahydro-1H-dibenz[2,3;6,7]oxepino-[4,5-c]pyrrole for 2 to 6 weeks after administration.

The invention also includes processes of manufacturing the pharmaceutical preparations which generally involves allowing the free base of trans-5-chloro-2-methyl-2,3,3a,12b-tetrahydro-1H-dibenz-[2,3:6,7]oxepino[4,5-c]pyrrole to react with pamoic acid to form a pamoate or hemipamoate salt.

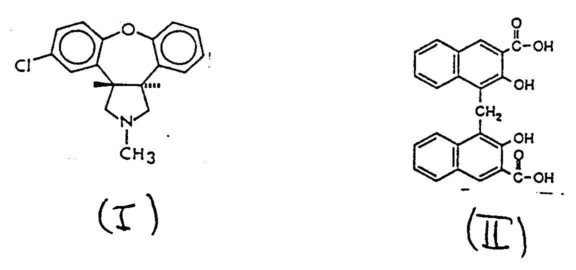

## Description

Technical Field. The invention relates to pharmaceutical preparations generally, and more specifically to a depot preparation useful in the treatment of various mental illnesses.

Background Art. The compound trans-5-chloro-2-methyl-2,3,3a,12b-tetrahydro-1H-dibenz[2,3;6,7]oxepino-[4,5-c]pyrrole and its preparation are described in U.S. Patent No. 4,145,434 to van den Burg, the contents of which are incorporated by this reference. The compound is described as having CNS-depressant activity and excellent antihistaminic and antiserotonin activities. Numerous acid addition salts of related compounds are described in this patent. However, the pamoate salt is not described.

### Disclosure of the Invention

Surprisingly it is found that the pamoate and hemipamoate salts of trans-5-chloro-2-methyl-2,3,3a,12b-tetrahydro-1H-dibenz-[2,3;6,7]oxepino-[4,5-c]pyrrole are very insoluble compounds having excellent properties for use as depot preparations.

The invention thus includes a pharmaceutical preparation comprising a depot preparation including trans-5-chloro-2-methyl-2,3,3a,12b-tetrahydro-1H-dibenz[2,3;6,7] oxepino-[4,5-c]pyrrole pamoate or hemipamoate. Such preparations will release therapeutically useful amounts of trans-5-chloro-2-methyl-2,3,3a,12b-tetrahydro-1H-dibenz[2,3;6,7] oxepino-[4,5-c]pyrrole after administration by injection.

The invention also includes processes of manufacturing the pharmaceutical preparations which generally involves allowing the free base of trans-5-chloro-2-methyl-2,3,3a,12b-tetrahydro-1H-dibenz-[2,3:6,7]oxepino [4,5-c]pyrrole to react with pamoic acid to form the pamoate or hemipamoate.

Once made, the compositions of the invention are useful in treating mammals, including humans, suffering from disease states which are susceptible to treatment by the free base generally. Such disease states include tension, excitation, anxiety, psychosis, schizophrenia. The compositions may also be used as antihistamines, and for their antiserotonin activities.

### Best Mode of the Invention

The free base trans-5-chloro-2-methyl-2,3,3a,12b-tetrahydro-1H-dibenz[2,3;6,7]oxepino-[4,5-c]pyrrole may be made as described in EXAMPLE IV of U. S. Patent No. 4,145,434 to van den Burg, the contents of the entire patent being incorporated by reference. The free base is also available from Organon International, bv of Oss, The Netherlands.

The chemical name of pamoic acid is 4,4'-methylenebis[3-hydroxy-2-napthalenecarboxylic acid]. It is readily commercially available, and is also known as "embonic acid".

In order to make the pamoate salt, trans-5-chloro-2-methyl-2,3,3a,12b-tetrahydro-1H-dibenz[2,3:6,7]oxepino[4,5-c]pyrrole is allowed to react with pamoic acid in an polar solvent until the pamoate is formed. Half the amount of pamoic acid is reacted to make the hemipamoate salt.

The pamoate salt spontaneously crystallizes with time at increased temperature (e.g. 50° C). This crystalline form -- which contains equal parts of pamoate salt and free base of the compound -- is prefered for use in the invention.

Whatever form the resulting salt takes, it may then be mixed with, for example, water, in order to make a pharmaceutical preparation for intramuscular injection. Other liquids which can be used with the pamoate compound to make depot preparations are vegetable oils, such as arachis or sesame oil.

Other pharmaceutical preparations into which the pamoate can be incorporated are implants including microspheres and microcapsules.

Although not necessary for the practice of the invention, other compounds such as phosphate salts, citric acid, and acetic acid or salts thereof; parabens; benzyl alcohol; and chlorobutanol may be included in the pharmaceutical preparations according to the invention to act as buffers, preservatives, and local anesthetics.

Suspending agents such as carboxymethylcelllulose or gelatin and surfactants such as Tween 20 or Tween 80 can be added to improve the suspension characteristics.

The dose of trans-5-chloro-2-methyl-2,3,3a,12b-tetrahydro-1H-dibenz[2,3:6,7]oxepino-[4,5-c]pyrrole pamoate contained within the pharmaceutical preparations of the invention are preferably chosen with a particular patient in mind. However, pharmaceutical preparations made according to the invention will typically contain sufficient trans-5-chloro-2-methyl-2,3,3a,12b-tetrahydro-1H-dibenz[2,3:6,7]oxepino-[4,5-c]pyrrole pamoate to release, in a sustained or prolonged manner, 2 to 4 mg daily into a subject are extremely useful in the practice of the invention. The ultimate dosage to provide relief for the patient depends, apart from individual characteristics, on the patient's weight, condition and age.

After intramuscular injection, the compound will typically release into an animal's blood stream for about four (from 2 to 6) weeks.

A method of providing therapy using the pharmaceutical preparation of the instant invention comprises administering it to a subject, such as a man, in need thereof. The treatment will generally be by intramuscular injection into the subject. The treatment may be continued for as long as necessary or desired.

The invention is further explained by the following illustrative EXAMPLES.

### EXAMPLE I

The free base of trans-5-chloro-2-methyl-2,3, 3a,12b-tetrahydro-1H-dibenz[2,3:6,7]oxepino-[4,5-c] pyrrole was obtained from Organon International, bv of Oss, The Netherlands. 2.91 grams of the free base was dissolved in 50 ml of dimethylformamide kept at 20° C. To this solution was added 3.95 g of pamoic acid (Janssen Chimica). The mixture was stirred for 20 hours, and the pamoate salt was obtained by adding 80 ml of water, the resulting precipitate was collected by filtration and slurried once with a 100 ml portion of acetone-hexane (1:1). The yellow solid was collected by filtration and dried in vacuo at 50° C for 48 hours; yield 5.52 g. The resulting pamoate salt had a water solubility of less than 0.2 mg/ml with a melting point of 224.3 to 254.1° C.

In a similar way were made the fumarate, 1-hydroxy-2-naphtoic acid and palmitic acid salts of trans-5-chloro-2-methyl-2,3,3a,12b-tetrahydro-1H-dibenz-[2,3;6,7]oxepino[4,5-c]pyrrole. The 1-hydroxy-2-naphtoic acid and palmitic acid salts were oils. The fumarate had a melting point of 185.5-187.5° C and water solubility of 1 mg / ml, which is not that desirable for a depot preparation.

### EXAMPLE II

A hemipamoate salt of the compound was made by the process of EXAMPLE I, but using only half (2 g) of the pamoic acid.

### EXAMPLE III

The pamoate salt of EXAMPLE I was stored for two weeks at 50° C, and crystals spontaneously formed. These crystals were less soluble and more stable than the amorphous yellow solid. Furthermore they were easier to segregate for micronization, and easier to form a suspension with.

### EXAMPLE V

A preparation for intramuscular injection includes:
10 mg/ml trans-5-chloro-2-methyl-2,3,3a,12b-tetrahydro 1H-dibenz[2,3:6,7]oxepino[4,5-c]pyrrole pamoate
10 mg/ml citric acid monohydrate
18 mg/ml disodium biphosphate·dihydrate
0.5 mg/ml polysorbate 80
5 mg/ml sodium carboxymethylcellulose
qsad 1 ml water

### EXAMPLE VI

A preparation for intramuscular injection includes:
50 mg/ml trans-5-chloro-2-methyl-2,3,3a,12b-tetrahydro 1H-dibenz[2,3:6,7]oxepino[4,5-c]pyrrole pamoate
10 mg/ml citric acid monohydrate
18 mg/ml disodium biphosphate·dihydrate
0.5 mg/ml polysorbate 80
5 mg/ml sodium carboxymethylcellulose
qsad 1 ml water

### EXAMPLE VII

A preparation for intramuscular injection includes:
100 mg/ml trans-5-chloro-2-methyl-2,3,3a,12b-tetrahydro 1H-dibenz[2,3:6,7]oxepino[4,5-c]pyrrole (crystal of EXAMPLE III)
10 mg/ml citric acid monohydrate
18 mg/ml disodium biphosphate·dihydrate
0.5 mg/ml polysorbate 80
5 mg/ml sodium carboxymethylcellulose
qsad 1 ml water
Reference herein to specific embodiments or examples should not be interpreted as limitations to the scope of the invention, which is defined by the appended claims.

## Claims

1. A pamoate or hemipamoate salt of trans-5-chloro-2-methyl-2,3,3a,12b-tetrahydro-1H-dibenz[2,3:6,7] oxepino[4,5-c]pyrrole.

2. Trans-5-chloro-2-methyl-2,3,3a,12b-tetrahydro-1H-dibenz[2,3:6,7]oxepino[4,5-c]pyrrole pamoate or hemipamoate for use in therapy.

3. Trans-5-chloro-2-methyl-2,3,3a,12b-tetrahydro-1H-dibenz[2,3:6,7[oxepino[4,5-c]pyrrole pamoate for use in the manufacture of a depot pharmaceutical preparation.

4. A depot pharmaceutical preparation comprising trans-5-chloro-2-methyl-2,3,3a,12b-tetrahydro-1H-dibenz[2,3:6,7]oxepino[4,5-c]pyrrole pamoate.

5. The depot preparation of claim 4 wherein the trans-5-chloro-2-methyl-2,3,3a,12b-tetrahydro-1H-dibenz[2,3:6,7]oxepino[4,5-c]pyrrole pamoate is present in crystalline form.

6. Use of trans-5-chloro-2-methyl-2,3,3a,12b-tetrahydro-1H-dibenz[2,3:6,7]oxepino[4,5-c]pyrrole pamoate or hemipamoate for the manufacture of a medicament having antipsychotic activity.

7. A process of manufacturing a pharmaceutical product comprising a pamoate or hemipamoate salt of trans-5-chloro-2-methyl-2,3,3a,12b-tetra-hydro-1H-dibenz-[2,3:6,7]oxepino[4,5-c]pyrrole, said process characterized in that the free base trans-5-chloro-2-methyl-2,3,3a,12b-tetrahydro-1H-dibenz-[2,3:6,7]oxepino[4,5-c]pyrrole is reacted with pamoic acid.

8. Trans-5-chloro-2-methyl-2,3,3a,12b-tetrahydro-1H-dibenz[2,3:6,7[oxepino[4,5-c]pyrrole hemipamoate for use in the manufacture of a depot pharmaceutical preparation.

9. A depot pharmaceutical preparation comprising trans-5-chloro-2-methyl-2,3,3a,12b-tetrahydro-1H-dibenz[2,3:6,7]oxepino[4,5-c]pyrrole hemipamoate.
